(19) Europäisches Patentamt
European Patent Office
Office européen des brevets

(11) Publication number: 0 431 799 A2

(12) # EUROPEAN PATENT APPLICATION

(21) Application number: 90312683.7

(22) Date of filing: 21.11.90

(51) Int. Cl.5: **A61K 31/52**, A61K 31/44, A61K 31/415, C07D 471/04

The title of the invention has been amended (Guidelines for Examination in the EPO, A-III, 7.3).

(30) Priority: 22.11.89 GB 8926417

(43) Date of publication of application: 12.06.91 Bulletin 91/24

(84) Designated Contracting States: AT BE CH DE DK ES FR GB GR IT LI LU NL SE

(71) Applicant: THE WELLCOME FOUNDATION LIMITED
Unicorn House 160 Euston Road
London NW1 2BP(GB)

(72) Inventor: Averett, Devron Randolph
2608 Lochmore Drive
Raleigh, North Carolina 27608(US)
Inventor: Daluge, Susan Mary
297 Azelea Drive
Chapel Hill, North Carolina 27514(US)

(74) Representative: Florence, Julia Anne et al
The Wellcome Research Laboratories, Group Patents & Agreements, Langley Court
Beckenham, Kent BR3 3BS(GB)

(54) **Use of nucleoside derivatives as cytotoxic agents.**

(57) Use of a compound of formula (I)

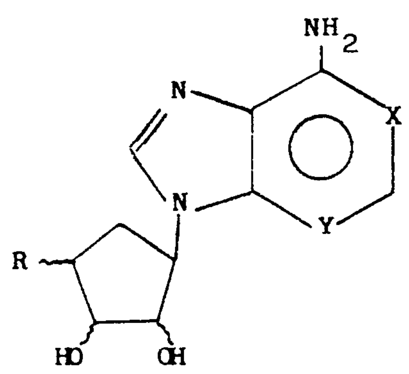

(I)

wherein R represents
a hydrogen atom; a $C_{1-4}$ alkyl group;
a group $COR^1$ wherein $R^1$ represents amino or $C_{1-4}$ alkoxy; or
a group $-CH_2R^2$ wherein $R^2$ represents a halogen atom,
$C_{1-4}$ alkylthio, or azido; and
X and Y each independently represent -CH- or -N;
or a physiologically acceptable salt, ester or other physiologically functional derivative thereof,
for the manufacture of a medicament for the treatment or prophylaxis of neoplastic growth in an animal.

EP 0 431 799 A2

## MEDICAMENTS

The present invention relates to the use of carbocyclic nucleoside derivatives as cytotoxic agents. In particular the present invention relates to the use of carbocyclic adenosine derivatives in the manufacture of medicaments for the treatment and/or prophylaxis of neoplastic growth.

Certain carbocyclic nucleoside derivatives have previously been reported to have antitumour activity and/or antiviral activity e.g. Neplanocin A, which has the structure:

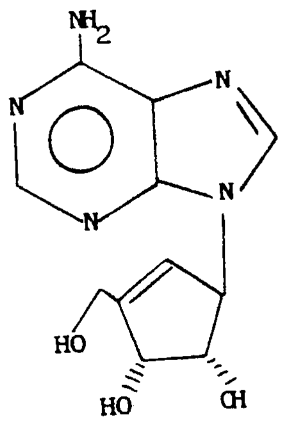

(Borcherding et al., J. Org. Chem., Vol. 52, No. 24, 1987).

Analogues of neplanocin A, of formula

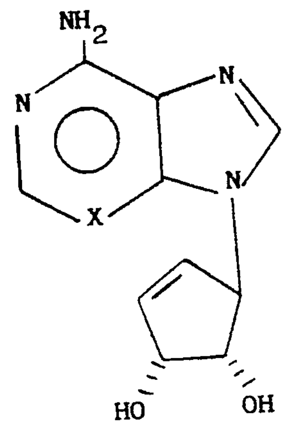

X - N or CH

are described by Hasobe et al. in Antimicrobial Agents and Chemotherapy, Nov. 1987, p1849-1851, and are said to have antiviral activity but reduced cytotoxicity.

Hayashi and Yaginuma (J. Antibiotics XXXIV, No. 6, p675-680) describe a number of derivatives prepared in the course of the structural elucidation of neplanocin A, including the cyclopentane derivative of the structure:

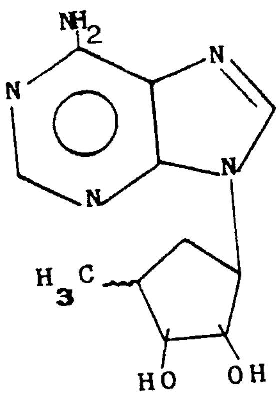

No biological activity is ascribed to this compound.

EPA 267878 discloses compounds of the formula:

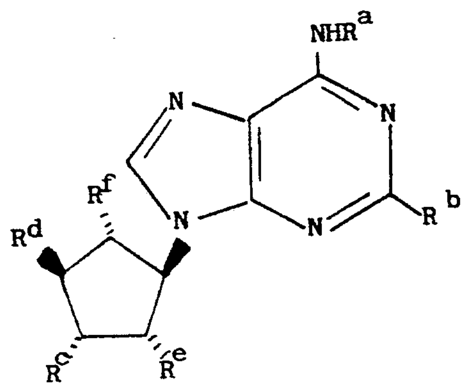

where $R^a$, $R^b$ and $R^f$ each represent inter alia hydrogen, $R^c$ and $R^e$ each represent inter alia hydroxy and $R^d$ represents inter alia lower alkylthiomethyl or -CONHR$^g$ where $R^g$ is inter alia lower alkyl. These compounds are said to be adenosine receptor agonists which may be useful in treating e.g. cardiovascular conditions and central nervous system conditions.

European Patent Application No. 368640, published after the priority date of the present application, discloses compounds of the formula:

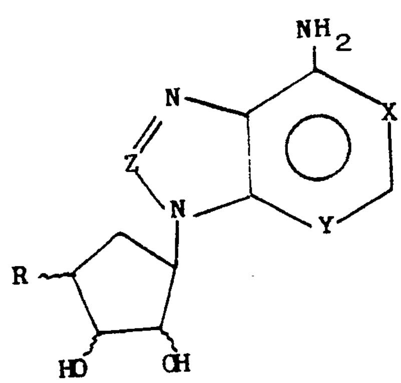

wherein R represents

a hydrogen atom; a $C_{1-4}$ alkyl group;

a group COR$^1$ wherein $R^1$ represents amino or $C_{1-4}$ alkoxy; or

a group -$CH_2R^2$ wherein $R^2$ represents a halogen atom (e.g. chlorine or bromine), $C_{1-4}$ alkylthio, or azido;

X and Y each independently represent -CH- or -N-; and

Z represents -CR$^3$- wherein $R^3$ is hydrogen or $C_{1-4}$ alkyl, or Z represents -N-; and their physiologically acceptable salts, esters and other physiologically functional derivatives, which compounds are said to have utility in the treatment of parasitic infections, eg. P.carinii pneumonia.

Before the publication of EPA 368640 it was surprisingly found that certain of the compounds described therein also have antineoplastic activity, in that they are able to inhibit the unregulated multiplication and proliferation of transformed cells (i.e. tumour causing cells). Such activity has been demonstrated against lymphocytic cells in the lymphocytic leukemia P388/0 test described hereinafter.

In a first aspect therefore the present invention provides the use of a compound of general formula (I).

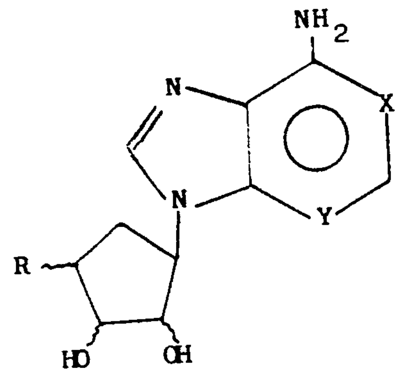

(I)

wherein R represents a hydrogen atom, a $C_{1-4}$ alkyl group;
a group $COR^1$ wherein $R^1$ represent amino or $C_{1-4}$ alkoxy;
or a group $-CH_2R^2$ wherein $R^2$ represents a halogen atom (e.g. chlorine or bromine), $C_{1-4}$ alkylthio, or azido; and
X and Y each independently represent -CH- or -N-; or a physiologically acceptable salt, ester or other physiologically functional derivative thereof;
for the manufacture of a medicament for the treatment or prophylaxis of neoplastic growth in an animal.

The animal requiring treatment or prophylaxis with a compound of formula (I) is usually a human or non-human mammal.

In another aspect the present invention provides a compound of general formula (I) or a physiologically acceptable salt, ester or other physiologically functional derivative thereof for use in the treatment and/or prophylaxis of neoplastic growth in an animal.

In a yet further aspect the present invention provides a method for the treatment or prophylaxis of neoplastic growth in an animal which comprises administering to said animal, a therapeutically or prophylactically effective amount of a compound of formula (I) or a physiologically acceptable salt, ester or other physiologically functional derivative thereof.

Particular examples of neoplastic growth requiring treatment or prophylaxis include lymphocytic and lymphoblastic leukemia, lymphoma and malignant tumours e.g. sarcomas, carcinomas eg. lung squamous carcinoma, lung small cell carcinoma and breast ductal carcinoma; adenocarcinomas eg. of the lung, colon and breast; melanomas; and hepatomas. Preferably the neoplastic growth is well-differentiated.

Preferred neoplastic growths which may be treated according to the present invention include lymphoblastic T-cell leukemia, hepatoma, and adenocarcinoma of the colon and lung, said neoplastic growths being preferably well-differentiated.

In the compound of formula (I) R is preferably hydrogen. X and Y are preferably both -N-.

Examples of physiologically acceptable salts of the compounds according to the invention include acid addition salts formed with organic carboxylic acids such as acetic, lactic, tartaric, maleic, citric, pyruvic, oxalic, fumaric, oxaloacetic, isethionic, lactobionic and succinic acids; organic sulfonic acids such as methanesulfonic, ethanesulfonic, benezenesulfonic and p-tolunesulfonic acids and inorganic acids such as hydrochloric, sulfuric, phosphoric and sulfamic acids.

Esters of compounds of formula (I) may be formed at either or both of the 2′- and 3′- hydroxyl groups, e.g. with a carboxylic or sulphonic acid. Alternatively the ester may bridge the 2′ and 3′-hydroxyl groups e.g. the 2′,3′-sulphite ester. Esters of formula (I) may act as pro-drugs, or may have activity in their own right.

Physiologically functional derivatives of compounds of formula (I) are derivatives which can be converted in the body into the parent compound. Such physiologically functional derivatives may also be referred to as "pro-drugs" or "bioprecursors". Physiologically functional derivatives of compounds (I) include in vivo hydrolysable esters.

It will be appreciated that the compounds of formula (I) may exist in various stereoisomeric forms and

the compounds of formula (I) as hereinbefore defined include all stereoisomeric forms and mixtures thereof, including enantiomers and racemic mixtures. The present invention includes within its scope the use of any such stereoisomeric form or mixture of stereoisomers, including the individual enantiomers of the compounds of formula (I) as well as wholly or partially racemic mixtures of such enantiomers.

A preferred configuration for the compounds of formula (I) is that represented by formula (IA):-

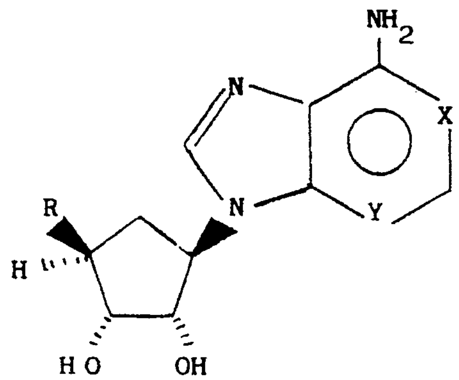

(IA)

A preferred compound for use according to the present invention is:-

(±)-(1R*,2S*,3R*)-3-(6-amino-9H-purin-9-yl)-1,2-cyclopentanediol;

Another preferred compound is:-

(±)-(1R*,2S*,3R*)-3-(4-amino-1H-imidazo[4,5-c]pyridin-1-yl)-1,2-cyclo pentanediol. This is believed to be a novel compound and as such forms a further feature of this invention.

A compound of formula (I) or a physiologically acceptable salt, ester or other physiologically functional derivative thereof may be administered alone or in combination with other drugs as part of a chemotherapeutic regimen for the treatment of neoplastic growth, or it may be administered as an adjunct to other forms of therapy. Thus for example a compound of formula (I) or a physiologically acceptable salt, ester or other physiologically functional derivative thereof may be administered alone or in combination with other drugs as a preparatory regimen intended to cause bone marrow ablation prior to autologous or allogeneic bone marrow transplantation for leukemia, lymphoma, myeloma or other malignancies, for example in an analogous manner to the administration of high doses of busulfan and cyclophosphamide prior to bone marrow transplantation for acute leukemia. (Santos, G.W, Bone Marrow Transplant, 1989 Jan;4 suppl. 1, 236-9)

The amount of a compound of formula (I) required for use in the treatment or prophylaxis of neoplastic growth e.g. lymphocytic leukemia, lymphoma or a malignant tumour, will depend inter alia on the route of administration, the age and weight of the patient and the nature and severity of the condition being treated and will ultimately be at the discretion of the attendant physician or veterinarian. In general, a suitable dose for administration to man is in the range of 0.1 to 100 mg. per kilogram bodyweight per day, for example from 1 mg/kg. to 40 mg/kg., per day particularly 5 to 15 mg/kg. per day. For administration by inhalation the dose may conveniently be in the range of 0.1 to 50 mg/kg/day, e.g. 1 to 10 mg/kg/day.

It will be appreciated that for administration to neonates, lower doses may be required.

For prophylactic treatment a compound of formula (I) or a physiologically acceptable salt, ester or other physiologically functional derivative thereof may also be given less frequently, e.g. as a single dose on alternate days, once or twice per week or once or twice per month. The dosage for prophylatic treatment will depend inter alia on the frequency of administration, and, where a depot preparation or controlled release formulation is used the rate of release of the active ingredient. Thus for once-weekly administration a suitable prophylactic dose is in the range 1 to 100 mg/kg, e.g. 5 to 50 mg/kg particularly 15 to 30 mg/kg.

For use according to the present invention a compound of formula (I) is preferably presented as a pharmaceutical formulation, comprising a compound of formula (I) or a physiologically acceptable salt, ester or other physiologically functional derivative thereof (hereinafter referred to as 'active compound') together with one or more pharmaceutically acceptable carriers therefor and optionally other therapeutic and/or prophylactic ingredients. The carrier(s) must be acceptable in the sense of being compatible with the other ingredients of the formulation and not deleterious to the recipient thereof.

An active compound may conveniently be presented as a pharmaceutical formulation in unit dosage form. A convenient unit dose formulation contains an active compound in an amount of from 25 mg to 100mg.

Pharmaceutical formulations include those suitable for oral, topical (including dermal, buccal and sublingual), rectal or parenteral (including subcutaneous, intradermal, intramuscular and intravenous), nasal and pulmonary administration e.g. by inhalation. The formulation may, where appropriate, be conveniently presented in discrete dosage units and may be prepared by any of the methods well known in the art of pharmacy. All methods include the step of bringing into association an active compound with liquid carriers or finely divided solid carriers or both and then, if necessary, shaping the product into the desired formulation.

Pharmaceutical formulations suitable for oral administration wherein the carrier is a solid are most preferably presented as unit dose formulations such as boluses, capsules or tablets each containing a predetermined amount of an active compound. A tablet may be made by compression or moulding, optionally with one or more accessory ingredients. Compressed tablets may be prepared by compressing in a suitable machine an active compound in a free-flowing form such as a powder or granules optionally mixed with a binder, lubricant, inert diluent, lubricating agent, surface-active agent or dispersing agent. Moulded tablets may be made by moulding an active compound with an inert liquid diluent. Tablets may be optionally coated and, if uncoated, may optionally be scored. Capsules may be prepared by filling an active compound, either alone or in admixture with one or more accessory ingredients, into the capsule shells and then sealing them in the usual manner. Cachets are analogous to capsules wherein an active compound together with any accessory ingredient(s) is sealed in a rice paper envelope. An active compound may also be formulated as dispersable granules, which may for example be suspended in water before administration, or sprinkled on food. The granules may be packaged e.g. in a sachet. Formulations suitable for oral administration wherein the carrier is a liquid may be presented as a solution or a suspension in an aqueous or non-aqueous liquid, or as an oil-in-water liquid emulsion.

Formulations for oral administration include controlled release dosage forms e.g. tablets wherein an active compound is formulated in an appropriate release - controlling matrix, or is coated with a suitable release - controlling film. Such formulations may be particularly convenient for prophylactic use.

Pharmaceutical formulations suitable for rectal administration wherein the carrier is a solid are most preferably presented as unit dose suppositories. Suitable carriers include cocoa butter and other materials commonly used in the art. The suppositories may be conveniently formed by admixture of an active compound with the softened or melted carrier(s) followed by chilling and shaping in moulds.

Pharmaceutical formulations suitable for parenteral administration include sterile solutions or suspensions of an active compound in aqueous or oleaginous vehicles. Injectible preparations may be adapted for bolus injection or continous infusion. Such preparations are conveniently presented in unit dose or multi-dose containers which are sealed after introduction of the formulation until required for use. Alternatively, an active compound may be in powder form which is constituted with a suitable vehicle, such as sterile, pyrogen-free water, before use.

An active compound may also be formulated as long-acting depot preparations, which may be administered by intramuscular injection or by implantation e.g. subcutaneously or intramuscularly. Depot preparations may include, for example, suitable polymeric or hydrophobic materials, or ion-exchange resins. Such long-acting formulations are particularly convenient for prophylactic use.

Formulations suitable for pulmonary administration via the buccal cavity are presented such that particles containing an active compound and desirably having a diameter in the range 0.5 to 7 microns are delivered into the bronchial tree of the recipient.

As one possibility such formulations are in the form of finely comminuted powders which may conveniently be presented either in a pierceable capsule, suitably of, for example, gelatin, for use in an inhalation device, or alternatively as a self-propelling formulation comprising an active compound, a suitable liquid or gaseous propellant and optionally other ingredients such as a surfactant and/or a solid diluent. Suitable liquid propellants include propane and the chlorofluorocarbons, and suitable gaseous propellants include carbon dioxide. Self-propelling formulations may also be employed wherein an active compound is dispensed in the form of droplets of solution or suspension.

Such self-propelling formulations are analogous to those known in the art and may be prepared by established procedures. Suitably they are presented in a container provided with either a manually-operable or automatically functioning valve having the desired spray characteristics; advantageously the valve is of a metered type delivering a fixed volume, for example, 25 to 100 microlitres, upon each operation thereof.

As a further possibility an active compound may be in the form of a solution or suspension for use in an atomiser or nebuliser whereby an accelerated airstream or ultrasonic agitation is employed to produce a fine droplet mist for inhalation.

Formulations suitable for nasal administration include presentations generally similar to those described above for pulmonary administration. When dispensed such formulations should desirably have a particle

diameter in the range 10 to 200 microns to enable retention in the nasal cavity; this may be achieved by, as appropriate, use of a powder of a suitable particle size or choice of an appropriate valve. Other suitable formulations include coarse powders having a particle diameter in the range 20 to 500 microns, for administration by rapid inhalation through the nasal passage from a container held close up to the nose, and nasal drops comprising 0.2 to 5% w/v of an active compound in aqueous or oily solution or suspension.

It should be understood that in addition to the aforementioned carrier ingredients the pharmaceutical formulations described above may include, as appropriate one or more additional carrier ingredients such as diluents, buffers, flavouring agents, binders, surface active agents, thickeners, lubricants, preservatives (including anti-oxidants) and the like, and substances included for the purpose of rendering the formulation isotonic with the blood of the intended recipient.

Therapeutic formulations for veterinary use may conveniently be in either powder or liquid concentrate form. In accordance with standard veterinary formulation practice, conventional water soluble excipients, such as lactose or sucrose, may be incorporated in the powders to improve their physical properties. Thus particularly suitable powders of this invention comprise 50 to 100% w/w, and preferably 60 to 80% w/w of the active ingredients(s), and 0 to 50% w/w and preferably 20 to 40% w/w of conventional veterinary excipients. These powders may either be added to animal feedstuffs, for example by way of an intermediate premix, or diluted in animal drinking water.

Liquid concentrates of this invention suitably contain a water-soluble compound of formula (I) or a salt thereof and may optionally include a veterinarily acceptable water-miscible solvent, for example polyethylene glycol, propylene glycol, glycerol, glycerol formal or such a solvent mixed with up to 30% v/v of ethanol. The liquid concentrates may be administered to the drinking water of animals.

In addition to being administered to an animal (eg. a human), a compound of formula (I) or a physiologically acceptable salt, ester, or other physiologically functional derivative thereof may also be used to treat blood or bone marrow ex vivo i.e. removed or isolated from an animal body, to remove malignant stem cells, eg. bone marrow erythroid stem cells or bone marrow granulocyte-macrophage stem cells for example by incubating the blood or bone marrow with a compound of formula (I) in an analogous manner to that described for 4-hydroperoxycyclophosphamide by Yeager, A.M. et al., N.Engl. J. Med., July 17 1986, 315 (3), 141-7, and such treatment forms a further aspect of this invention.

Compounds of formula (I) and physiologically acceptable salts, esters and other physiologically functional derivatives thereof may be prepared according to methods known in the art, for example European Patent Application No. 368640, which methods include:

A) hydroxylation of a compound of formula (II):

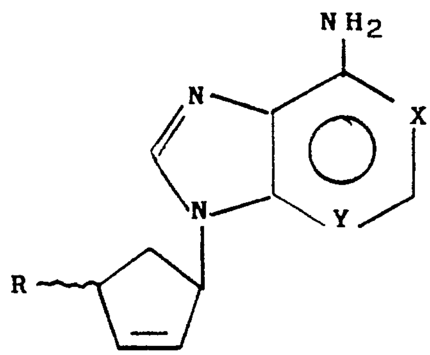

(II)

wherein R, X, and Y are as defined for formula (I)

B) amination of a compound of formula (III):

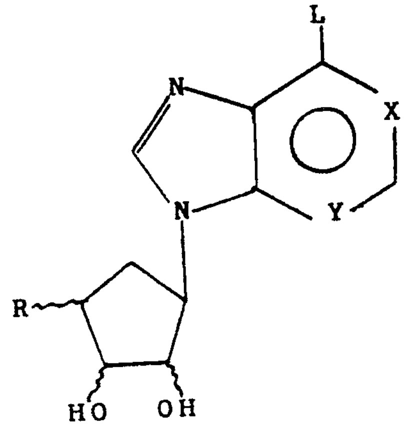

(III)

wherein R, X and Y are as defined for formula (I) and L is a leaving group replaceable by $-NH_2$;

C) to prepare compounds wherein R represents halomethyl, reaction of a compound of formula (IV):

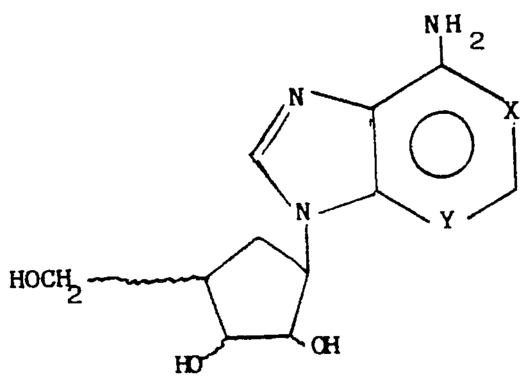

(IV)

wherein X and Y are as defined for formula (I), with a halogenating agent.

D) interconversion of one compound of formula (I) into a different compound of formula (I) e.g. preparation of compounds of formula (I) wherein R represents methyl by reduction of a compound of formula (I) wherein R represents halomethyl.

Hydroxylation according to process (A) may be effected using a catalytic amount of osmium tetroxide, in the presence of a tertiary amine oxide, e.g. N-methylmorpholine N-oxide monohydrate. The reaction will generally be carried out in a solvent, preferably an aqueous organic solvent such as an aqueous alcohol e.g. t-butyl alcohol. The reaction temperature may conveniently be between 0° and 150°.

Compounds of formula (II) wherein Z represents -CH- may be prepared by amination of the corresponding compound of formula (V):

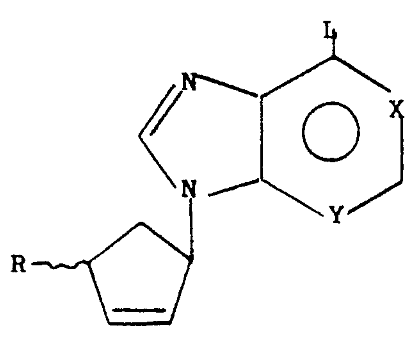

(V)

wherein R, L, X and Y are as hereinbefore defined. The amination may be carried out using ammonia, or by

reacting with hydrazine followed by reduction with Raney Nickel as described for process (B).

Compounds of formula (V) may themselves be prepared by reaction of a compound of formula (VI):

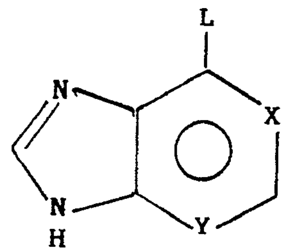

(VI)

with a cyclopentene derivative of formula (VII):

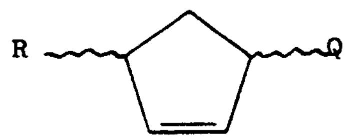

(VII)

(wherein Q is a leaving group eg. a halogen atom)
which may for example be prepared in situ from cyclopentene (or an appropriately substituted derivative thereof) with e.g. N-bromosuccinimide.

Alternatively compounds of formula (V) may be prepared by reaction of a compound of formula (VIII)

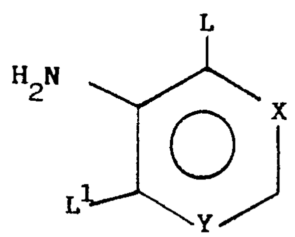

(VIII)

wherein X and Y are as hereinbefore defined and L and $L^1$ each represent leaving groups replaceable by $-NH_2$, with an appropriately substituted 3-aminocyclopentene derivative, in the presence of a solvent, e.g. an alcohol, and a base e.g. triethylamine, at a temperature in the range 50° to 150°, to give a compound of formula (IX):

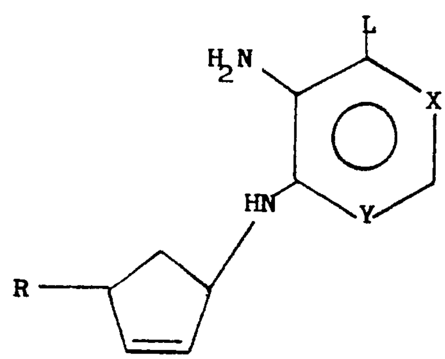

(IX)

followed by cyclisation to a compound of formula (V). The cyclisation may be carried out, e.g., by reacting a compound of formula (IX) with formic acid or a reactive formic acid derivative, e.g. triethylorthoformate or diethoxymethyl acetate, in a solvent such as a dimethylacetamide or acetonitrile at an elevated temperature, preferably at 75-90°. This reaction is conveniently effected by the addition of a catalytic amount of a strong

anhydrous acid, e.g., ethanesulfonic acid in which case lower temperatures, e.g., 25°C., are used. A suitable catalytic amount of the acid may be in the range 0.1 to 2.5%, conveniently around 2%.

Amination of a compound of formula (III) according to process (B) may be effected by reaction with ammonia, or with hydrazine followed by reduction with Raney Nickel in the presence or absence of solvent, conveniently at a temperature in the range of 0° to 100°C. Solvents which may be employed in the reaction include water and organic solvents, e.g. alcohols. When X represents -N- and Y represents -CH-it is preferred to carry out the amination using hydrazine and Raney Nickel.

A specific embodiment of process B comprises aminating a compound of formula (III) wherein R is a group COR[1] where R[1] represents $C_{1-4}$ alkoxy. In addition to replacement of the leaving group L, the alkoxy group R[1] is replaced by $NH_2$, to give a compound of formula (I) wherein R is $-CONH_2$.

Compounds of formula (III) may be prepared by hydroxylation of a compound of formula (V), using the general methods described for process (A) above. Alternatively, compounds of formula (III) may be prepared by cyclisation of a compound of formula (X):

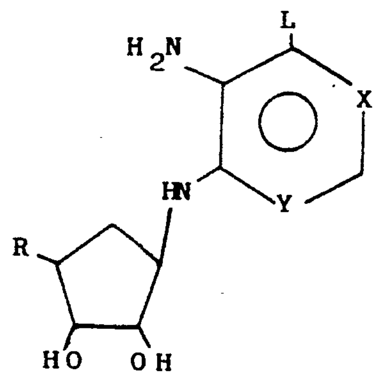

(X)

using similar conditions to those described for the cyclisation of compounds (IX).

Compounds of formula (X) may themselves be prepared by reaction of a compound of formula (VIII) with a compound of formula (XI):

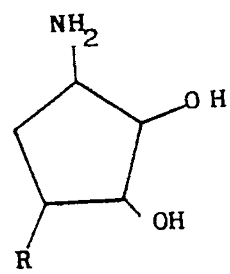

(XI)

wherein R is as hereinbefore defined. Preparation of compounds of formula (XI) may be effected by hydroxylation of a protected 5-(R)-3-amino-cyclopentene derivative, as described for process (A) followed by removal of the protecting group. The amino protecting group may be selected from the conventional protecting groups known in the art. Advantageously the protecting group is a phthaloyl group. Compounds of formula (XI) wherein R is a group COR[1] and R[1] is $C_{1-4}$ alkoxy may be prepared by the method described by S. Daluge and R. Vince (J.Org.Chem, 1978,12, 2311).

Alternatively, a compound of formula (X) may be prepared by reduction of the corresponding nitro compound for example using a metal halide eg. stannous chloride, in an aqueous acid eg. hydrochloric acid. The nitro compound may itself be prepared by reacting a compound of formula (XI) with an appropriately substituted pyridine or pyrimidine, such as 3-nitro-4-ethoxy-pyridine, in the presence of a base eg. triethylamine and an organic solvent such as an alcohol eg. ethanol. This method is particularly convenient for the preparation of compounds (X) where X represents -N- and Y represents -CH- (see also J.Med.Chem 1982, 25, 626, J.A.Montgomery et al).

Preparation of compounds of formula (I) according to process (C) may be effected by reaction of a compound of formula (IV) with a halogenating agent, in the presence of a solvent, conveniently at a

temperature in the range -15° to 30°. Solvents which may be employed include amides, e.g. hexamethylphosphoramide or N, N-dimethylformamide. The halogenating agent may be for example thionyl chloride, oxalyl chloride or phosphorus pentachloride. Reaction with the halogenating agent will generally be followed by neutralisation, e.g. with ammonium hydroxide. Compounds of formula (IV) may be prepared by the method described in J.Am.Chem.Soc. 1969, 91, 3075.

Conversion of a compound of formula (I) wherein R represents halomethyl into a corresponding compound wherein R represents methyl may be effected using a reducing agent such as tributyl tin in the presence of a free radical initiator e.g.α,α-azobisisobutyrylnitrile. The reaction may conveniently be effected in the presence of an anhydrous solvent e.g. an ether such as tetrahydrofuran.

Specific examples of the preparation of compounds of formula (I) according to the above methods can be found in published European Patent Application No. 368640.

The preparation of the novel compound (±)-(1R*,2S*,3R*)-3-(4-amino-1H-imidazo[4,5-c]pyridin-1-yl)-1,2-cyclopentanediol is illustrated below:-

EXAMPLE 1

a) (±)-(1R*,2S*,3R*)-3-[(3-Nitro-4-pyridyl)amino]-1,2-cyclopentanediol

(±)-(1R*,2S*,3R*)-3-Amino-1,2-cyclopentanediol (J.P.Whitten, J.R.McCarthy, and M.R.Whalon, J.Org.Chem. 1985, 50, 4399) (2.49g, 17.4mmol), 4-ethoxy-3-nitropyridine hydrochloride (J.B.Campbell, et al, J.Heterocyclic Chem. 1986, 23, 669)(3.61g, 17.4mmol),triethylamine (4.9mL), and ethanol (12ml) were refluxed under nitrogen for 26 hours. The reaction mixture was cooled and the precipitate which formed was collected and washed with ethanol. Recrystallisation from ethanol gave title compound as yellow powder (3.27g, 79%); mp174-175°C; $^1$H-NMR(DMSO-$d_6$)δ: 9.01(s,1,pyridineH-2),8.24(d,J=6.25,1,pyridineH-6), 8.15-(d,J=7.0,1,NH),7.07(d,J=6.25,1,pyridineH-5)4.96(d,J=5.9,1,OH),4.6-0(d,J=3.9,1,OH),4.0-3.75(m,3,3CH),2.4-1.4(m,H,2$CH_2$).

Anal. Calcd for $C_{10}H_{13}N_3O_4$ : C,50.21;H,5.48;N,17.56. Found: C,50.28; H,5.51; N,17.46.

b) (±)-(1R*,2S*,3R*)-3-[(2-Chloro-3-amino-4-pyridyl)amino]-1,2-cyclopentanediol

(±)-(1R*,2S*,3R*)-3-[(3-Nitro-4-pyridyl)amino]-1,2-cyclopentanediol (2.39g, 10.0mmol) and concentrated hydrochloric acid (24ml) were heated to 90-100°C (oil bath) while stannous chloride dihydrate (11.3g, 50meq) was added in several portion over five minutes. After an additional 30 minutes at ca. 100°C the pale yellow solution was diluted with water (50ml) and evaporated to dryness. The residue was redissolved in $H_2O$ (40ml) and the pH adjusted to 9 with concentrated ammonium hydroxide (ca. 14ml). The mixture was filtered with Celite and the filter pad washed with warm ethanol. The filtrate-wash was evaporated to dryness and the residue chromatographed on silica gel. Title compound was eluted with 20-25% methanol-chloroform as a white solid foam (1.70g, 70%), suitable in purity for the next step. Crystallisation of such a sample from ethanol gave title compound as white crystals; mp 213-216°C; $^1$H-NMR(DMSO-$d_6$)δ: 7.40-(d,J=5.2,1, pyridine H-6), 6.47 (d,J=5.2, 1, pyridine H-5), 5.62 (brd, J=5.9,1,NH), 4.82(s,2,$NH_2$), 4.69-(d,J=5.3,1,OH), 4.55(d,J=4.7,1,OH), 3.91(m,1,CH); 3.70-3.45 (m,2,2CH), 2.3-1.2 (m,H,2$CH_2$).

Anal. Calcd for $C_{10}H_{14}N_3ClO_2$ : C,49.29; H,5.79; N,17.24; Cl,14.55. Found : C49.31; H,5.82; N,17.18; Cl,14.58

c) (±)-(1R*,2S*,3R*)-3-(4-Chloro-1H-imidazo[4,5-c]pyridin-1-yl)-1,2-cyclopentanediol

(±)-(1R*,2S*,3R*)-3-[(2-Chloro-3-amino-4-pyridyl)amino]-1,2-cyclopentanediol (2.28g, 9.36mmol)-,triethylorthoformate (50ml), and ethanesulfonic acid (1.13g, 10.3mmol) were stirred for 3 days. The mixture was filtered to remove inorganic salts from the previous step. The filtrate was evaporated to a dark syrup (2.6g) which was chromatographed on silica gel. Elution with 2% methanol-chloroform gave an off-white solid (1.63g) which $^1$H-NMR showed to be a mixture of title compound and 1′ 2′ formate esters of title compound. This solid was stirred in IN ammonium hydroxide (20ml)-dioxane (20ml) overnight. The resulting solution was evaporated to dryness and the residue crystallised from acetonitrile to give title compound as white clusters of needles (0.60g, 27%);mp 209-213°C; $^1$H-NMR (DMSO-$d_6$)δ: 8.63 (s,1H-2),8.14 (d,J=5.6,1,H-6),7.75 (d,J=5.6,1H-7),5.10(d,J=6.85, 1,OH), 4.81) (d,J=3.35,1,OH), 4.76(m,1,H-3), 4.22-(m,1,H-2), 4.00(m,1,H-1), 2.4-1.4(m,4,2$CH_2$).

Anal.Calcd for $C_{11}H_{12}N_3ClO_2$ : C,52.08; H,4.77; N,16.56; Cl, 13.98. Found : C,52.07; H,4.80; N,16.61;

Cl,14.03.

d) (±)-(1R*,2S*,3R*)-3-(4-Amino-1H-imidazo[4,5-c]pyridin-1-yl)-1,2-cyclopentanediol

(±)-(1R*,2S*,3R*)-3-(4-Chloro-1H-imidazo[4,5-c]pyridin-1-yl)-1,2-cyclopentanediol (0.66g, 2.1 mmol) and hydrazine hydrate (Aldrich Chemical Co., 98%, 25ml) were heated at 95°C (oil bath) for 1.5 hours. The solution was evaporated to a dark yellow solid foam. Oxygen-free water (20ml) and Raney Nickel powder, 50% slurry in water, washed until neutral (Aldrich Chemical Co., 2ml wet) were added and the mixture heated at 90-100°C under nitrogen with vigourous stirring for 45 minutes. The hot mixture was filtered through a Celite pad and the pad washed with hot ethanol (3X20ml). The filtrate-wash was evaporated to dryness and the residual glass (0.57g) chromatographed on silica gel. Title compound was eluted with 25% methanol-chloroform as a white solid (329mg, 67%), $^1$H-NMR identical with a sample recrystallised from ethanol-water; mp 255-258°C; $^1$H-NMR(DMSO-$d_6$)δ: 8.16 (s,1,H-2), 7.64(d,J=5.8,1,H-6), 6.79 (d,J=5.8,1,H-7), 6.12 (br s,2,$NH_2$), 5.05 (d,J=6.85,1,OH), 4.77(d,J=3.4,1,OH),4.595 (m,1,H-3), 4.20(m,1,H-2), 3.99(m,1,H-1), 2.4-1.4 (m,H,2$CH_2$); mass spectrum (70 eV,EI) : 234(M), 135(B+2H), 134(B+H); UVλmax at pH1; : 263-(9500), 268sh; at pH7 : 263.5 (9860); at pH13 : 266 (10,150).

Anal. Calcd for $C_{11}H_{14}N_4O_2$.0.8NaCl : C,47.02; H,5.02; N,19.94; Cl, 1.09. Found : C,46.76;H,4.93; N,19.71; Cl,9.89.

Chemotherapeutic Data

The invention is further illustrated by the following biological test data, which however do not constitute any limitation of the present invention:

A. Antineoplastic Activity Data for Lymphocytic Leukemia P388/0 Test.

The tests employed for evaluating the antitumour activity of compounds of the present invention are essentially those used in the Tumor Panel of the Developmental Therapeutics Program, Divison of Cancer Treatment National Cancer Institute, A Goldin, et al., Methods in Cancer Research, Vol. XVI, p. 165, Academic Press (1979). Some modifications in dose level and schedule have been made to increase the testing efficiency.

Male CD2-$F_1$ mice, weighing 20 ± 3 g, were used for this test. Control and test animals were injected intraperitioneally with a suspension of ~$10^6$ viable P388 tumor cells on day 0. In each test, several dose levels which bracketed the $LD_{20}$ of the compound were evaluated; each dose level group contained six animals. The test compounds were prepared either in physiologic saline containing 0.05% Tween 80 or distilled water containing 5% dextrose and were administered (a) orally, (b) intravenously or (c) intraperitoneally according to the indicated schedule relative to the day of tumour implant. Doses were on a mg/kg basis according to individual animals' body weights. The day of death for each animal was recorded, the median identified for each group and the percent increase in life span (%ILS) was calculated from the ratios of median survival time for treated to control groups. The criterion for activity is % ILS greater than or equal to 20%.

Test Compound:

(±)-(1R*,2S*,3R*)-3-(6-amino-9H-purin-9-yl)-1,2-cyclopentanediol.

| Results | | | |
|---|---|---|---|
| Dose | Schedule | %Increase in Lifespan | Toxic at: |
| a) Oral Dosing | | | |
| 15mg/kg<br>25mg/kg | Day 1,5,9<br>Day 1,2,3,4,5 | + 25<br>+ 35 | 35 mg/kg<br>35 mg/kg |
| b) Intravenous Dosing | | | |
| 75/mg/kg<br>75/mg/kg | Day 1,5,9<br>Day 1,2,3,4,5 | + 30<br>+ 35 | >175mg/kg<br>150mg/kg |
| c) Intraperitoneal Dosing | | | |
| 40mg/kg<br>40mg/kg | Day 1,5,9<br>Day 1,2,3,4,5 | + 20<br>+ 15 | 80mg/kg<br>60mg/kg |

## Claims

1. Use of a compound of formula (I)

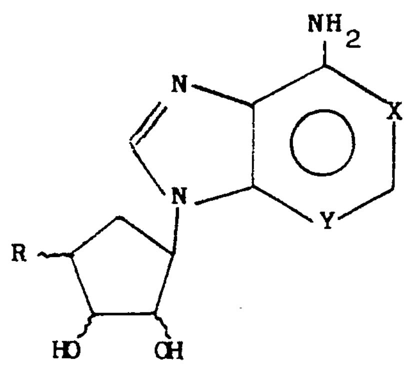

(I)

wherein R represents
a hydrogen atom; a $C_{1-4}$ alkyl group;
a group $COR^1$ wherein $R^1$ represents amino or $C_{1-4}$ alkoxy; or
a group $-CH_2R^2$ wherein $R^2$ represents a halogen atom,
$C_{1-4}$ alkylthio, or azido; and
X and Y each independently represent -CH- or -N-;
or a physiologically acceptable salt, ester or other physiologically functional derivative thereof,
for the manufacture of a medicament for the treatment or prophylaxis of neoplastic growth in an animal.

2. Use according to claim 1 of a compound of formula (I) wherein R represents a hydrogen atom.

3. Use according to claim 1 or claim 2 of a compound of formula (I) wherein X and Y each represent -N-.

4. Use according to any of claims 1 to 3 of a compound of formula (I) which has the configuration (IA)

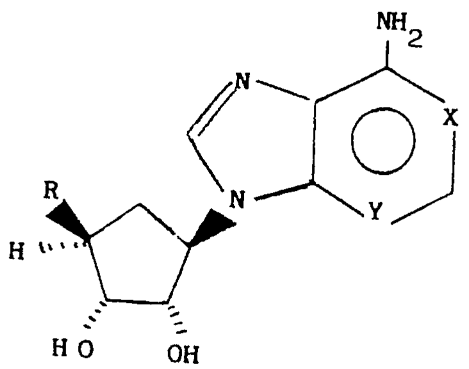

(IA)

wherein R, X and Y are as defined in any of claims 1 to 3.

5. Use according to any of claims 1 to 4 of a compound of formula (I) which is (±)-(1R*,2S*,3R*)-3-(6-amino-9H-purin-9-yl)-1,2-cyclopentanediol.

6. Use according to any of claims 1 to 5 wherein the neoplastic growth is a lymphocytic leukemia, lymphoma or malignant tumour.

7. Use according to claim 6 wherein the malignant tumour is a sarcoma, carcinoma, adenocarcinoma, hepatoma or melanoma.

8. Use of a compound (I) as defined in claim 1 for the manufacture of a medicament for promoting bone marrow ablation.

9. Use according to any of claims 1 to 8 wherein the mammal is a human.

10. A pharmaceutical composition for the treatment of neoplastic growth comprising a compound of formula (I) as defined in any of claims 1 to 5, or a physiologically acceptable salt, ester or other physiologically functional derivative thereof together with a pharmaceutically acceptable carrier therefor.

11. Method of treating cells ex-vivo which comprises contacting said cells with a compound of formula (I) as defined in any of claims 1 to 5.

12. Method according to claim 11 wherein the cells are blood or bone marrow cells.

13. Compound of formula (I) which is (±)-(1R*, 2S*, 3R*)-3-(4-amino-1H-imidazo[4,5-c]pyridin-1-yl)-1,2-cyclopentanediol, or a physiologically acceptable salt, ester or other physiologically functional derivative thereof.

14. A pharmaceutical composition comprising a compound of formula (I) as defined in claim 13 or a physiologically acceptable salt, ester or other physiologically functional derivative thereof, together with a pharmaceutically acceptable carrier therefor.

15. A process for preparing a compound of formula (I) as defined in claim 13 which comprises:

A) hydroxylation of a compound of formula (II):

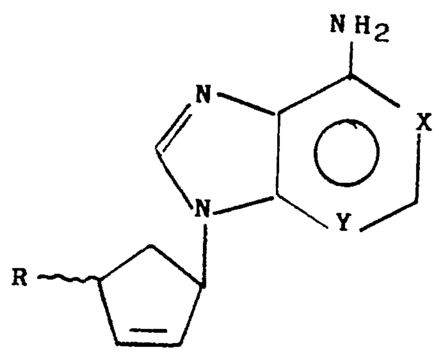

(II)

wherein R represents hydrogen, X is -N- and Y is -CH-; or

B) amination of a compound of formula (III):

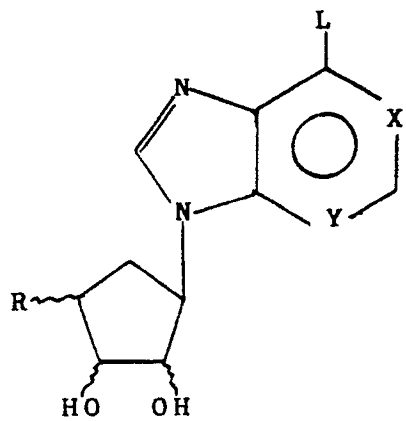

(III)

wherein R represents hydrogen, X is -N-, Y is -CH- and L is a leaving group replaceable by $-NH_2$;
followed where necessary or desired by formation of a physiologically acceptable salt, ester or other physiologically functional derivative thereof.